Europäisches Patentamt

European Patent Office

Office européen des brevets

(19)

(11) Veröffentlichungsnummer: **0 340 487 B1**

# (12) EUROPÄISCHE PATENTSCHRIFT

(45) Veröffentlichungstag der Patentschrift: **09.12.92**

(51) Int. Cl.⁵: **C07C 15/08**, C07C 7/14

(21) Anmeldenummer: **89106389.3**

(22) Anmeldetag: **11.04.89**

(54) **Verfahren zur Gewinnung von p-Xylol mit einer Reinheit von mindestens 99,5 %.**

(30) Priorität: **05.05.88 DE 3815324**

(43) Veröffentlichungstag der Anmeldung:
**08.11.89 Patentblatt 89/45**

(45) Bekanntmachung des Hinweises auf die
Patenterteilung:
**09.12.92 Patentblatt 92/50**

(84) Benannte Vertragsstaaten:
**DE FR GB**

(56) Entgegenhaltungen:
**DE-A- 3 703 646**
**FR-A- 2 114 772**
**US-A- 2 769 852**

(73) Patentinhaber: **Krupp Koppers GmbH**
**Altendorfer Strasse 120**
**W-4300 Essen 1(DE)**

(72) Erfinder: **Puppel, Günter, Dipl.-Ing.**
**Kampstrasse 4**
**W-4273 Wulfen(DE)**

**Beschreibung**

Die Erfindung betrifft ein Verfahren zur Gewinnung von p-Xylol mit einer Reinheit von mindestens 99,5 % aus einem Vorprodukt mit hohem p-Xylolgehalt, wobei das Vorprodukt im flüssigen Zustand in eine inerte Flüssigkeit, vorzugsweise Wasser, eingeleitet wird, deren Temperatur unterhalb des Schmelzpunktes des reinen p-Xylols liegt, worauf die sich unter Rühren abscheidende Kristallphase in einer Trenneinrichtung von der inerten Flüssigkeit abgetrennt und anschließend in einer Schmelzeinrichtung aufgeschmolzen wird, aus der die p-Xylolkristalle mit der gewünschten Reinheit abgezogen werden, während das in der inerten Flüssigkeit nach Abtrennung des p-Xylols noch vorhandene Kohlenwasserstoffgemisch durch Phasenscheidung aus der Flüssigkeit ausgeschieden und daran anschließend in den der Herstellung des Vorproduktes dienenden Prozeß zurückgeführt wird.

Ein derartiges Verfahren wird in der nicht zum Stande der Technik gehörenden DE-A1-37 03 646 beschrieben. Mit Hilfe dieses Verfahrens gelingt es, unter Verwendung eines in den bereits vorhandenen Produktionsanlagen erzeugten Vorproduktes ein p-Xylol mit einer Reinheit von mindesten 99,5 % zu erzeugen, ohne daß die vorhandenen Produktionsanlagen umgebaut werden müssen und ohne daß es deshalb zu Betriebsstillständen kommt.

Aus der US-A-2 769 852 ist ferner ein Verfahren zur Abtrennung von Xylolgemischen oder einzelnen Xylolisomeren aus diese Verbindungen enthaltenden Einsatzprodukten bekannt, bei dem das Einsatzprodukt im flüssigen Zustand bei Temperaturen unterhalb des Schmelzpunktes des Isomeren in eine inerte Flüssigkeit eingeleitet wird, aus der sich die abscheidenden, die Isomeren enthaltenden Kristalle abgetrennt werden. Im Gegensatz zum erfindungsgemäßen Verfahren wird hierbei jedoch von einem Einsatzprodukt ausgegangen, das lediglich zwischen 1 und 25 Gew.-% p-Xylol enthält und außerdem erfolgt die Auskristallisierung durch Tiefkühlung.

Die vorliegende Erfindung betrifft die weitere Ausgestaltung des Verfahrens nach der DE-Al-37 03 646, wobei das Verfahren in folgenden Punkten verbessert werden soll:

1. Vereinfachung des Verfahrensablaufes;

2. Anwendbarkeit des Verfahrens zur Gewinnung eines p-Xylols mit einer Reinheit von mehr als 99,5 % und

3. Anwendbarkeit des Verfahrens bei Verwendung eines Vorproduktes, dessen p-Xylolgehalt kleiner als 99 % ist.

Dies wird bei einem Verfahren der eingangs genannten Art erfindungsgemäß dadurch erreicht, daß die als Kälteträger verwendete inerte Flüssigkeit unter Rotation in einem Mischbehälter mit einem Vorprodukt zusammengeführt wird, dessen p-Xylolgehalt mindesten 97 % beträgt, wobei das resultierende Gemisch aus dem Unterteil des Mischbehälters abgezogen und in einer Filtereinrichtung bis auf einen Kristallanteil von 30 bis 70 % eingedickt und daran anschließend unmittelbar der Trenneinrichtung zugeführt und dort bis auf eine Temperatur zwischen 7 und 13°C erwärmt wird, wobei bei Verwendung eines Vorproduktes mit einem p-Xylolgehalt von <99 % die Menge des in den der Herstellung des Vorproduktes dienenden Prozeß zurückgeführten Kohlenwasserstoffgemisches entsprechend erhöht wird.

Diese Arbeitsweise stellt gegenüber dem älteren Verfahren eine Vereinfachung dar, weil hierbei das aus der Filtereinrichtung abgezogene Gemisch unmittelbar in die Trenneinrichtung eingeleitet werden kann und der beim älteren Verfahren als Zwischenstufe vorgesehene, mit einem Rührwerk ausgestattete Vorlagebehälter entfällt. Außerdem werden bei der erfindungsgemäßen Arbeitsweise die Verfahrensbedingungen im Mischbehälter dadurch verbessert, daß die inerte Flüssigkeit mit dem Vorprodukt unter Rotation zusammengeführt wird. Die durch die Rotation der Flüssigkeit bewirkte Trombenbildung ermöglicht es, das stark auftreibende Kristallagglomerat kontinuierlich aus dem Mischbehälter nach unten abzuziehen. Die erforderliche Rotation kann dabei dadurch bewirkt werden, daß die inerte Flüssigkeit tangential in den Mischbehälter eingeführt wird, in dem zusätzlich ein Rührwerk installiert ist.

Die Erhöhung der Reinheit des gewonnenen p-Xylols über den Wert von 99,5 % hinaus kann dadurch erreicht werden, daß die abgetrennten p-Xylolkristalle in der Trenneinrichtung auf eine Temperatur im Bereich zwischen 7 und 13°C erwärmt werden, so daß sie mit einer entsprechend erhöhten Temperatur die Trenneinrichtung verlassen und der Schmelzeinrichtung zugeführt werden. Die Erwärmung kann durch geeignete Flüssigkeiten, wie z.B. vorgewärmtes Wasser, vorgewärmtes Einsatzprodukt oder eine andere Flüssigkeit, die sich nachträglich leicht vom gereinigten p-Xylol abtrennen läßt, erfolgen. Es können hierfür aber auch Dampf oder erhitztes Gas, wie z.B. Stickstoff, verwendet werden.

Beim älteren Verfahren ist vorgesehen, daß das als Ausgangsmaterial dienende Vorprodukt einen p-Xylolgehalt von mindestens 99 % aufweist. Es hat sich jedoch gezeigt, daß auch von einem Vorprodukt mit einem niedrigeren p-Xylol-Gehalt ausgegangen werden kann. Allerdings erhöht sich in diesem Falle die Menge des Kohlenwasserstoffgemisches, die in den der Herstellung des Vorproduktes dienenden Prozeß zurückgeführt werden muß. Bei Verwendung eines Vorproduktes mit einem p-Xylol-Gehalt von 99 % beträgt die zurückgeführte Menge ca. 6 %des Einsatzproduktes. Unter den gleichen Prozeßbedingungen vergrößert sich die Rückführmenge bei einem Vorprodukt mit 98 % p-Xylol auf ca. 13 % des Einsatzproduktes und bei einem Vorprodukt mit 97 % p-Xylol auf ca. 2o % des Einsatzproduktes. Es muß deshalb jeweils vom Betreiber der der Herstellung des Vorproduktes dienenden Anlage beurteilt werden, welche Fahrweise für ihn am wirtschaftlichsten ist.

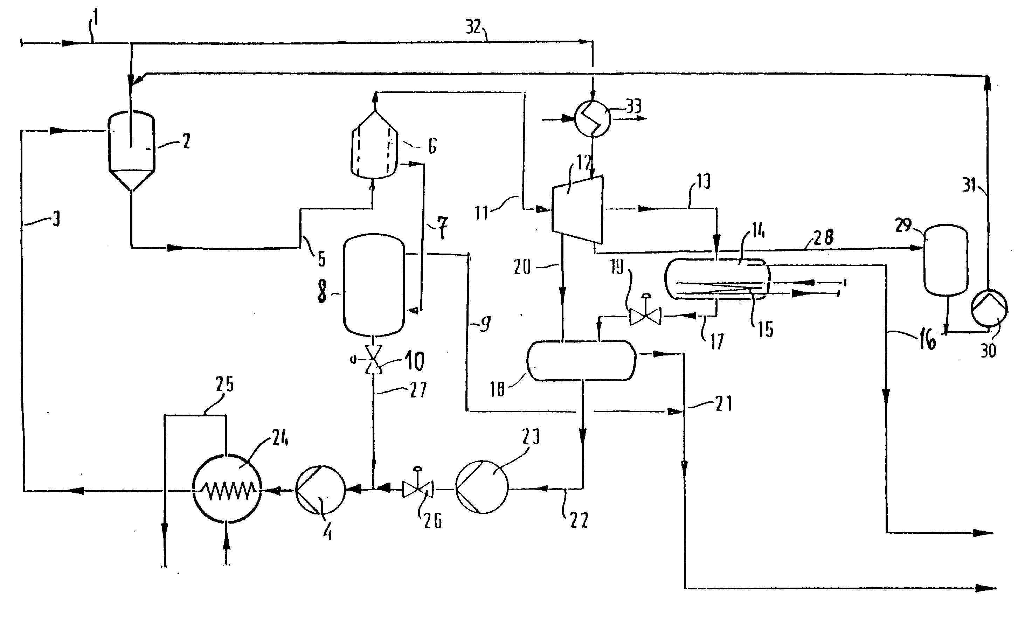

Weitere Einzelheiten der erfindungsgemäßen Arbeitsweise sollen nachfolgend an Hand des in der Abbildung dargestellten Fließschemas erläutert werden. Das Fließschema zeigt dabei selbstverständlich nur alle für die Verfahrenserläuterung unbedingt notwendigen Apparate und Anlagenteile.

Das aus der im Fließschema nicht dargestellten, vorgeschalteten Produktionsanlage stammende Vorprodukt wird über die Leitung 1 im flüssigen Zustand in den Mischbehälter 2 eingeleitet. Die erforderliche inerte Flüssigkeit, deren Temperatur unterhalb des Schmelzpunktes des reinen p-Xylols liegt, wird dem Mischbehälter 2 über die Leitung 3 zugeführt und tangential in diesen eingeleitet, so daß die inerte Flüssigkeit im Mischbehälter 2 unter Rotation mit dem Vorprodukt vermischt wird. Als inerte Flüssigkeit im Sinne der Erfindung ist hierbei eine Flüssigkeit zu verstehen, in der das p-Xylol keine oder nur eine ganz geringe Löslichkeit aufweist. Als Beispiele hierfür werden genannt: Wasser und Gemische aus Wasser und bestimmten Alkoholen, wie beispielsweise Methanol, Ethanol und Glykol. Die Temperatur der inerten Flüssigkeit muß, wie bereits gesagt wurde, bei der Einleitung des flüssigen Vorproduktes unterhalb des Schmelzpunktes des reinen p-Xylols, d.h. unterhalb 13,26°C, liegen. Vorzugsweise wird man mit einer Flüssigkeitstemperatur zwischen 0 und 12°C arbeiten.

Die durch die Rotation der inerten Flüssigkeit im Mischbehälter 2 bewirkte Trombenbildung ermöglicht es, stark auftreibende Kristallagglomerate kontinuierlich von unten aus dem Mischbehälter 2 abzuziehen und über die Leitung 5 von unten in die Filtereinrichtung 6 einzuleiten. Hier wird das Kristall-Flüssigkeitsgemisch bis auf einen Kristallanteil von 3o bis 7o % eingedickt und anschließend über die Leitung 11 unmittelbar der Trenneinrichtung 12 zugeführt, die in diesem Falle als Zentrifuge ausgebildet ist. Die Trenneinrichtung 12 kann dabei, wie bereits weiter oben erwähnt wurde, so ausgerüstet sein, daß in ihr eine Erwärmung der von der Flüssigkeit abgetrennten p-Xylolkristalle bis auf eine Temperatur im Bereich zwischen 7 und 13°C möglich ist. Die erwärmten p-Xylolkristalle werden aus der Trenneinrichtung 12 abgezogen und gelangen anschließend über die Leitung 13 in die Schmelzeinrichtung 14, die mit der dampfbeheizten Heizschlange 15 versehen ist. Mittels dieser Heizschlange 15 werden die Kristalle in der Schmelzeinrichtung 14 wieder aufgeschmolzen. Etwaige inerte Flüssigkeitsreste scheiden sich dabei ab und sammeln sich als schwerere Phase am Boden der Schmelzeinrichtung 14, so daß die geschmolzenen Kristalle aus dem Oberteil der Schmelzeinrichtung 14 über die Leitung 16 abgezogen werden können. Hierbei handelt es sich um das gewünschte Reinprodukt, dessen p-Xylol-Gehalt mindestens 99,5 % beträgt.

Die sich am Boden der Schmelzeinrichtung 14 ansammelnden Reste der inerten Flüssigkeit werden über die Leitung 17 in den Filtratbehälter 18 abgezogen. In der Leitung 17 ist dabei das Ventil 19 installiert, so daß dieser Flüssigkeitsabzug so eingestellt werden kann, daß die Trennschicht zwischen dem geschmolzenen p-Xylol und der inerten Flüssigkeit in der Schmelzeinrichtung 14 auf konstantem Niveau gehalten wird. In den Filtratbehälter 18 wird über die Leitung 2o auch die von der Trenneinrichtung 12 kommende Flüssigkeit eingeleitet. Im Filtratbehälter 18 können durch Phasenscheidung die in der inerten Flüssigkeit enthaltenen Kohlenwasserstoffe abgetrennt werden. Diese scheiden sich als leichtere Phase an der Flüssigkeitsoberfläche ab und können über die Leitung 21 abgezogen werden. Hierbei handelt es sich im wesentlichen um ein Gemisch aus o- und m-Xylol sowie Ethylbenzol mit niedrigem p-Xylolgehalt. Dieses Gemisch kann deshalb in den der Herstellung des Vorproduktes dienenden Prozeß zurückgeführt werden. Die Menge des zurückgeführten Gemisches richtet sich dabei, wie weiter oben erläutert wurde, nach der Reinheit des eingesetzten Vorproduktes.

In den Filtratbehälter 8 wird die in der Filtereinrichtung 6 abgetrennte Flüssigkeit über die Leitung 7 eingeleitet. Die hierbei gegebenenfalls mitgeführten Kohlenwasserstoffe scheiden sich im Filtratbehälter 8 als leichtere Phase an der Flüssigkeitsoberfläche ab und können über die Leitung 9 abgezogen und mit den Kohlenwasserstoffen in der Leitung 21 vereinigt werden. In Abweichung von der Darstellung im Fließschema ist es gegebenenfalls auch möglich, die aus dem Filtratbehälter 8 abgezogene leichte Phase über die Leitung 9 mit in den Filtratbehälter 18 einzuleiten.

Die von den Kohlenwasserstoffen befreite inerte Flüssigkeit wird über die Leitung 27 aus dem Filtratbehälter 8 abgezogen und mit der Pumpe 4 dem Wärmeaustauscher 24 zugeführt. Das Ventil 1o dient dabei der Regelung dieses Abzuges. Der Wärmeaustauscher 24 ist an den Kühlmittelkreislauf 25 angeschlossen, so daß die inerte Flüssigkeit im Warmeaustauscher 24 die erforderliche Wiederabkühlung erfährt, bevor sie über die Leitung 3 zum Mischbehälter 2 zurückgeführt wird.

In die Leitung 27 mündet die vom Filtratbehälter 18 kommende Leitung 22. Durch diese Leitung wird die sich am Boden des Filtratbehälters 18 ansammelnde, von Kohlenwasserstoffen befreite inerte Flüssigkeit abgezogen und mittels der Pumpe 23 zur Leitung 27 zurückgeführt. Das Ventil 26 dient dabei der Regelung dieses Flüssigkeitsstromes. In Abweichung von der Darstellung im Fließschema kann diese Rückführung auch in den Filtratbehälter 8 erfolgen.

Falls für die Erwärmung der p-Xylolkristalle in der Trenneinrichtung 12 ein Teilstrom des zum Einsatz gelangenden Vorproduktes verwendet werden soll, wird dieser Teilstrom über die Leitung 32 von dem Strom in der Leitung 1 abgezweigt. Bei Verwendung eines Vorproduktes mit einem p-Xylolgehalt von 99 % kann dieser Teilstrom beispielsweise etwa 25 % der Einsatzproduktmenge betragen. Im Wärmeaustauscher 33 erfährt der über Leitung 33 abgezogene Teilstrom die erforderliche Erwärmung und gelangt danach in die Trenneinrichtung 12. Die dort bei der Erwärmung der p-Xylolkristalle anfallende Filtratflüssigkeit wird in diesem Falle separat über die Leitung 28 aus der Trenneinrichtung 12 abgezogen und gelangt in die Pumpenvorlage 29. Von dort wird sie mit der Pumpe 3o über die Leitung 31 zur Leitung 1 zurückgefördert und dem Einsatzprodukt zugesetzt.

Eine Kristallabscheidung beim Herunterkühlen der im Kreislauf geführten inerten Flüssigkeit im Wärmeaustauscher 24 kann dadurch vermieden werden, daß der Flüssigkeit in den Leitungen 22 und 27 etwas Xylolgemisch mit einem geringen p-Xylolgehalt zugemischt wird, so daß der p-Xylolgehalt des in der Flüssigkeit gelösten Xylols so weit abgesenkt wird, daß der Kristallisationsbeginn unterhalb der Austrittstemperatur der gekühlten inerten Flüssigkeit aus dem Wärmetauscher 24 liegt.

Einer Emulsionsbildung, wie sie beispielsweise in der Trenneinrichtung 12 oder den Pumpen entstehen kann, wird durch den Einbau von handelsüblichen Einrichtungen zum Brechen von Emulsionen in den jeweils nachgeschalteten Leitungen begegnet.

Nachfolgend soll die Wirkungsweise des erfindungsgemäßen Verfahrens noch an Hand eines Ausführungsbeispiels erläutert werden:
Hierbei gelangte ein Vorprodukt mit einem p-Xylolgehalt von 99 % zum Einsatz. Dieses Vorprodukt wurde mit einer Temperatur von 3o°C in den Mischbehälter 2 eingeleitet. Als inerte Flüssigkeit wurde Wasser verwendet, das mit einer Temperatur von ca. 2°C tangential in den Mischbehälter 2, der zusätzlich mit einem Rührer ausgerüstet war, eingeführt wurde. Das aus dem Mischbehälter 2 abgezogene Wasser-Kristallgemisch wies einen Kristallgehalt von 7 - 8 % auf und wurde in der Filtereinrichtung 6 bis auf einen Kristallanteil von 3o % entwässert. Dieser Kristallbrei wurde mit einer Temperatur von ca. 6°C kontinuierlich einer Zentrifuge zugeführt, die in diesem Falle als Trenneinrichtung 12 fungierte. Ohne zusätzliche Erwärmung der dort abgeschiedenen p-Xylolkristalle wurde ein Endprodukt mit einem p-Xylolgehalt von 99,5 % erreicht.

Eine weitere Erhöhung der Reinheit des anfallenden Endproduktes konnte dadurch erreicht werden, daß ein Teilstrom des eingesetzten Vorproduktes in der Größenordnung von ca. 25 % zur Erwärmung der p-Xylolkristalle in der Zentrifuge herangezogen wurde. Dieser Teilstrom wurde mit einer Temperatur von ca. 45°C in die Zentrifuge eingeleitet. Die Reinheit des anfallenden Endproduktes konnte dadurch bis auf einen p-Xylolgehalt von > 99,8 % gesteigert werden.

**Patentansprüche**

1. Verfahren zur Gewinnung von p-Xylol mit einer Reinheit von mindestens 99,5 % aus einem Vorprodukt mit hohem p-Xylolgehalt, wobei das Vorprodukt im flüssigen Zustand in eine inerte Flüssigkeit, vorzugsweise Wasser, eingeleitet wird, deren Temperatur unterhalb des Schmelzpunktes des reinen p-Xylols liegt, worauf die sich unter Rühren abscheidende Kristallphase in einer Trenneinrichtung von der inerten Flüssigkeit abgetrennt und anschließend in einer Schmelzeinrichtung aufgeschmolzen wird, aus der die p-Xylolkristalle mit der gewünschten Reinheit abgezogen werden, während das in der inerten Flüssigkeit nach Abtrennung des p-Xylols noch vorhandene Kohlenwasserstoffgemisch durch Phasenscheidung aus der Flüssigkeit abgeschieden und daran anschließend in den der Herstellung des Vorproduktes dienenden Prozeß zurückgeführt wird, dadurch gekennzeichnet, daß die als Kälteträger verwendete inerte Flüssigkeit unter Rotation in einem Mischbehälter (2) mit einem Vorprodukt zusammengeführt wird, dessen p-Xylolgehalt mindestens 97 % beträgt, wobei das resultierende Gemisch aus dem Unterteil des Mischbehälters (2) abgezogen und in einer Filtereinrichtung (6) bis auf einen Kristallanteil von 30 bis 70 % eingedickt und daran anschließend unmittelbar der Trenneinrichtung (12) zugeführt und dort bis auf eine Temperatur zwischen 7 und 13°C erwärmt wird, wobei bei Verwendung eines Vorproduktes mit einem p-Xylolgehalt von < 99 % die Menge des in den der Herstellung des Vorproduktes dienenden Prozeß zurückgeführten Kohlenwasserstoffgemisches entsprechend erhöht wird.

2. Verfahren nach Anspruch 1, dadurch gekennzeichnet, daß zur Erwärmung der abgetrennten p-Xylolkristalle in der Trenneinrichtung (12) sowohl vorgewärmtes Wasser, vorgewärmtes Einsatzprodukt oder eine andere Flüssigkeit, die sich nachträglich leicht vom gereinigten p-Xylol abtrennen läßt, als auch Dampf oder erhitztes Gas, wie beispielsweise Stickstoff, verwendet werden.

3. Verfahren nach den Ansprüchen 1 und 2, dadurch gekennzeichnet, daß die Menge des in den der Herstellung des Vorproduktes dienenden Prozeß zurückgeführten Kohlenwasserstoffgemisches zwischen 6 und 20 % des eingesetzten Vorproduktes beträgt.

## Claims

1. Process for the recovery of p-xylene having a purity of at least 99.5 % from a precursor having a high p-xylene content, the precursor being introduced in the liquid state into an inert liquid, preferably water, the temperature of which is below the melting point of pure p-xylene, whereupon the crystal phase being formed with stirring is separated off from the inert liquid in a separating device and is then melted in a melting device from which the p-xylene crystals having the desired purity are withdrawn, while the hydrocarbon mixture still present in the inert liquid following the removal of the p-xylene is eliminated from the liquid by phase separation and is then returned to the process serving to prepare the precursor characterised in that the inert liquid used as coolant is combined with a precursor with rotation in a mixing vessel (2), the p-xylene content of which precursor is at least 97 %, the resulting mixture being withdrawn from the lower part of the mixing vessel (2), thickened in a filtration device (6) to a crystal content of 30 to 70 %, fed, following this, directly to the separating device (12) and warmed there to a temperature between 7 and 13°C, the amount of the hydrocarbon mixture returned to the process serving in the preparation of the precursor being correspondingly increased when a precursor having a p-xylene content of <99 % is used.

2. Process according to Claim 1, characterised in that, to heat the p-xylene crystals separated off in the separating device (12), prewarmed water, prewarmed feedstock or another liquid, which can be subsequently easily separated from the purified p-xylene, or alternatively steam or heated gas, such a for example nitrogen, is used.

3. Process according to Claims 1 and 2, characterised in that the amount of the hydrocarbon mixture returned to the process serving for the preparation of the precursor is between 6 and 20 % of the precursor used.

## Revendications

1. Procédé pour l'obtention de p-xylène d'une pureté d'au moins 99,5 % à partir d'un avant-produit d'une teneur élevée en p-xylène, cet avant-produit étant introduit à l'état liquide dans un liquide inerte, de préférence de l'eau, dont la température se situe au-dessous du point de fusion du p-xylène pur, après quoi la phase cristalline qui se sépare par agitation est séparée du liquide inerte dans un dispositif séparateur et fondue ensuite dans un dispositif de fusion d'où l'on soutire avec la pureté désirée les cristaux de p-xylène, tandis que le mélange hydrocarboné encore présent dans le liquide inerte après séparation du p-xylène est séparé du liquide par séparation des phases et renvoyé ensuite dans le processus servant à la préparation de l'avant-produit, caractérisé par le fait que le liquide inerte utilisé comme frigo-porteur est, par rotation dans un récipient de mélange (2), délivré avec un avant-produit dont la teneur en p-xylène est de 97 % au moins, le mélange résultant étant soutiré de la partie inférieure du récipient (2) de mélange et épaissi dans un dispositif de filtration (6) jusqu'à présenter une fraction cristalline de 30 à 70 % et est ensuite délivré directement au dispositif séparateur (12) et y est chauffé jusqu'à une température comprise entre 7 et 13°C, tandis qu'en utilisant un avant-produit d'une teneur en p-xylène inférieure à 99 %, on augmente de façon correspondante la quantité du mélange hydrocarboné renvoyée dans le processus servant à la préparation de l'avant-produit.

2. Procédé selon la revendication 1, caractérisé par le fait que pour chauffer les cristaux de p-xylène séparés dans le dispositif séparateur (2), on utilise aussi bien de l'eau préchauffée, du produit de mise en oeuvre préchauffé ou un autre liquide se laissant séparer facilement ultérieurement du p-xylène purifié, que de la vapeur ou du gaz chauffé, tel que de l'azote, par exemple.

3. Procédé selon les revendications 1 et 2, caractérisé par le fait que la quantité du mélange hydrocarboné renvoyé dans le processus servant à la préparation de l'avant-produit est comprise entre 6 et 20 % de l'avant-produit

**EP 0 340 487 B1**

mis en oeuvre,